# EUROPEAN PATENT APPLICATION

(11) **EP 4 428 226 A1**
(43) Date of publication of application: **11.09.2024**
(21) Application number: 22885467.5
(22) Date of filing: 14.09.2022
(51) Int. Cl.: C12N 1/21, C12P 13/08, C07K 14/195, C07K 14/21, C12R 1/15, C12R 1/19

(54) **RECOMBINANT MICROORGANISM CONSTRUCTED BASED ON LYSINE EFFLUX PROTEIN AND METHOD FOR PRODUCING LYSINE**

(30) Priority: 01.11.2021 CN 202111283285
(71) Applicant: Tianjin Institute of Industrial Biotechnology, Chinese Academy of Sciences, Tianjin 300308 (CN)
(72) Inventor: SUN, Jibin, Tianjin 300308 (CN); LIU, Jiao, Tianjin 300308 (CN); ZHENG, Ping, Tianjin 300308 (CN); SUN, Guannan, Tianjin 300308 (CN); ZHOU, Wenjuan, Tianjin 300308 (CN); NI, Xiaomeng, Tianjin 300308 (CN); CHEN, Jiuzhou, Tianjin 300308 (CN)
(74) Representative: Weickmann & Weickmann PartmbB
(86) International application number: PCT/CN2022/118751
(87) International publication number: WO 2023/071580

(57) **Abstract**

Provided are a recombinant microorganism constructed based on a lysine efflux protein, a method for constructing the recombinant microorganism, a lysine producing strain, and a method for producing lysine. The polypeptide having the sequence as shown in SEQ ID NO: 4 or SEQ ID NO: 6 can promote the extracellular discharge of lysine, and two novel lysine efflux proteins are provided for the transformation of a lysine high-producing strain. A recombinant microorganism is constructed based on the polypeptide having the sequence as shown in SEQ ID NO: 4 or SEQ ID NO: 6. The recombinant microorganism has significantly increased lysine yield and sugar-acid conversion rate, and is suitable for large-scale industrial production of lysine.

## Description

### PRIORITY RIGHT AND RELATED APPLICATION

The present disclosure claims the benefit of a priority of Chinese Patent Application No. 202111283285.0, filed on November 1, 2021, entitled "RECOMBINANT MICROORGANISM CONSTRUCTED BASED ON LYSINE EFFLUX PROTEIN AND METHOD FOR PRODUCING LYSINE", the entire contents of which including the appendices are incorporated herein by reference.

### TECHNICAL FIELD

The present disclosure belongs to the field of biotechnology and genetic engineering technology, and specifically relates to a recombinant microorganism constructed based on a lysine efflux protein, a method for constructing the recombinant microorganism, a lysine producing strain, and a method for producing lysine.

### BACKGROUND

L-lysine (lysine for short) is an important ingredient in human and animal nutrition and is widely used in industries such as food, feed, pharmaceuticals, and cosmetics. With growing market demands, lysine promises great economic potential. With continuous modifications of producing strains and the advancement of process engineering technology, the microbiological fermentation has been widely used in the industrial production of most amino acids such as lysine because of its good economic and environmental benefits.

Breeding high-yield fermentative microorganisms is the key to production by the fermentation method. Mutation breeding is mainly used at first to obtain high-yield strains. However, mutation breeding has shortcomings, such as heavy workload, difficulty in controlling directions of mutations, difficulty in accumulating multiple forward mutations, and reverse mutations of the resulting strains. With the advancement of recombinant DNA technology, metabolic engineering has begun to be applied in the construction process of high-yield strains, greatly shortening the cycles of strain modifications. For metabolically engineered recombinant bacteria, after the rate-limiting reaction is enhanced and the competitive metabolic pathways are eliminated or weakened, the target products can be produced more quickly in cells.

However, studies have found that excessive intracellular lysine concentrations will restrict the final yield of high-yield strains. Therefore, since the discovery of the first amino acid efflux protein - LysE (lysine efflux protein), the modification method of reducing the intracellular lysine concentration by an efflux protein to increase the yield of recombinant bacteria has attracted much attention.

LysE is an L-lysine efflux protein derived from *Corynebacterium glutamicum.* While this protein can efflux L-lysine^{[1]}, it can also efflux L-arginine, L-histidine, and L-ornithine^{[2-4]}. Moreover, it effluxes L-lysine and L-arginine at basically comparable rates and has very low substrate specificity, so it can hardly achieve specific and efficient efflux of L-lysine in L-lysine producing bacteria. The other efflux protein is an LysO family protein (also referred to as YbjE protein) derived from *Escherichia coli,* which can efficiently efflux L-lysine and significantly improve the lysine yield, and its abilities to efflux L-arginine and improve the L-arginine yield are remarkably lower as compared to L-lysine^{[5-6]}. The above two proteins are currently the sole proteins that have actually been identified as having the lysine efflux function. Although more than 20,000 proteins derived from different strains have been annotated as lysine efflux proteins of the LysO family, whether they really have the lysine efflux function is still unknown. Therefore, the development of lysine efflux proteins capable of efficiently exporting lysine out of cells to reduce the intracellular lysine concentration and increase the lysine yield is an important issue to be urgently addressed in the field of microbial fermentation.

### References:

[1] Bellmann A, Vrljić M, Pátek M, Sahm H, Krämer R, Eggeling L. Expression control and specificity of the basic amino acid exporter LysE of Corynebacterium glutamicum. Microbiology (Reading). 2001;147(Pt 7):1765-1774.
[2] CN102747025A
[3] Wu H, Tian D, Fan X, et al. Highly Efficient Production of l-Histidine from Glucose by Metabolically Engineered Escherichia coli. ACS Synth Biol. 2020; 9(7):1813-1822.
[4] CN102947460A
[5] CN101243177B
[6] Pathania A, Sardesai AA. Distinct Paths for Basic Amino Acid Export in Escherichia coli: YbjE (LysO) Mediates Export of L-Lysine. J Bacteriol. 2015; 197(12):2036-2047.

### SUMMARY

### Technical Problem

In view of the problems existing in the prior art, for example, a need to develop more lysine efflux proteins having a high efficiency in exporting lysine so as to improve the yield of lysine produced by microorganisms, the present disclosure provides a recombinant microorganism constructed based on a polypeptide having a sequence as set forth in SEQ ID NO: 4 or SEQ ID NO: 6. The present disclosure has found that the polypeptide having a sequence as set forth in SEQ ID NO: 4 or SEQ ID NO: 6 is capable of promoting efflux of lysine out of cells and effectively increasing the yield of lysine produced by microbiological fermentation, which provides two novel lysine efflux proteins for modifications of lysine high-producing strains.

### Solution to Problem

(1) A recombinant microorganism, wherein the recombinant microorganism has an enhanced activity and/or expression level of a lysine efflux protein as compared to a wild-type microorganism; and the lysine efflux protein is at least one selected from the group consisting of (i) to (iv):
   (i) a polypeptide comprising an amino acid sequence as set forth in SEQ ID NO: 4;
   (ii) a polypeptide mutant comprising an amino acid sequence as set forth in SEQ ID NO: 4, the polypeptide mutant having one or more amino acids substituted, repeated, deleted or added at one or more positions of the sequence as set forth in SEQ ID NO: 4, and having an activity of the lysine efflux protein;
   (iii) a polypeptide comprising an amino acid sequence as set forth in SEQ ID NO: 6; and
   (iv) a polypeptide mutant comprising an amino acid sequence as set forth in SEQ ID NO: 6, the polypeptide mutant having one or more amino acids substituted, repeated, deleted or added at one or more positions of the sequence as set forth in SEQ ID NO: 6, and having an activity of the lysine efflux protein.
(2) The recombinant microorganism according to (1), wherein the lysine efflux protein is encoded by a polynucleotide shown in any one of (v) to (vii):
   (v) a polynucleotide encoding a polypeptide of the amino acid sequence as set forth in SEQ ID NO: 4 or SEQ ID NO: 6;
   (vi) a polynucleotide comprising a nucleotide sequence as set forth in SEQ ID NO: 10 or SEQ ID NO: 12; and
   (vii) a polynucleotide having at least 90%, optionally at least 95%, preferably at least 97%, more preferably at least 98%, most preferably at least 99% to the sequence as set forth in SEQ ID NO: 10 or SEQ ID NO: 12 and encoding a polypeptide having an activity of the lysine efflux protein.
(3) The recombinant microorganism according to (1) or (2), wherein the recombinant microorganism is derived from a microorganism of *Escherichia, Erwinia, Serratia, Providencia, Enterobacteria, Salmonella, Streptomyces, Pseudomonas, Brevibacterium* or *Corynebacterium;*
   optionally, the recombinant microorganism is derived from *Corynebacterium glutamicum* or *Escherichia coli;*
   preferably, the recombinant microorganism is derived from *Corynebacterium glutamicum* ATCC 13032, *Corynebacterium glutamicum* ATCC 13869, *Corynebacterium glutamicum* ATCC 14067 or a derivative strain of any one thereof.
(4) The recombinant microorganism according to any one of (1) to (3), wherein the recombinant microorganism contains a mutated gluconate repressor GntR1; preferably, the mutation is a mutation of arginine at position 102 to alanine.
(5) A method for constructing the recombinant microorganism according to any one of (1) to (4), comprising a step of enhancing an activity of a lysine efflux protein in a microorganism and/or a step of enhancing an expression level of a lysine efflux protein in a microorganism; wherein the lysine efflux protein is at least one selected from the group consisting of (i) to (iv):
   (i) a polypeptide comprising an amino acid sequence as set forth in SEQ ID NO: 4;
   (ii) a polypeptide mutant comprising an amino acid sequence as set forth in SEQ ID NO: 4, the polypeptide mutant having one or more amino acids substituted, repeated, deleted or added at one or more positions of the sequence as set forth in SEQ ID NO: 4, and having an activity of lysine efflux protein;
   (iii) a polypeptide comprising an amino acid sequence as set forth in SEQ ID NO: 6; and
   (iv) a polypeptide mutant comprising an amino acid sequence as set forth in SEQ ID NO: 6, the polypeptide mutant having one or more amino acids substituted, repeated, deleted or added at one or more positions of the sequence as set forth in SEQ ID NO: 6, and having an activity of the lysine efflux protein.
(6) The method according to (5), wherein the method further comprises a step of mutating a gluconate repressor GntR1 of the recombinant microorganism; preferably, the mutation is a mutation of arginine at position 102 to alanine.
(7) Use of the recombinant microorganism according to any one of (1) to (4), or a recombinant microorganism constructed by the method according to (5) or (6) in the production of lysine.
(8) A lysine producing strain, wherein the lysine producing strain is the recombinant microorganism according to any one of (1) to (4), or a recombinant microorganism constructed by the method according to (5) or (6).
(9) The lysine producing strain according to (8), wherein the lysine producing strain is derived from a microorganism of *Escherichia, Erwinia, Serratia, Providencia, Enterobacteria, Salmonella, Streptomyces, Pseudomonas, Brevibacterium* or *Corynebacterium;*
   optionally, the lysine producing strain is derived from *Corynebacterium glutamicum* or *Escherichia coli;*
   preferably, the lysine producing strain is derived from *Corynebacterium glutamicum* ATCC 13032, *Corynebacterium glutamicum* ATCC 13869, *Corynebacterium glutamicum* ATCC 14067 or a derivative strain of any one thereof.
(10) A method for producing lysine, comprising a step of producing lysine by fermenting the recombinant microorganism according to any one of (1) to (4), the lysine producing strain according to (8) or (9), or a recombinant microorganism constructed by the method according to (5) or (6);
   optionally, the method further comprising a step of isolating the lysine from a reaction solution for producing the lysine by fermentation.

### Advantageous Effects of the Invention

In some embodiments, the present disclosure provides use of a polypeptide having a sequence as set forth in SEQ ID NO: 4 or SEQ ID NO: 6 for effluxing lysine out of cells of a microorganism. The polypeptide having the sequence as set forth in SEQ ID NO: 4 or SEQ ID NO: 6, as a novel lysine efflux protein, is capable of effectively promoting transfer of lysine out of the cells of the microorganism, in particular has high substrate specificity to efflux of lysine out of cells, and is capable of effectively improving the efflux efficiency of lysine, thereby increasing the yield of lysine produced by microbiological fermentation, which provides a novel and efficient lysine efflux protein for modifications of the lysine high-producing strains.

In some embodiments, the recombinant microorganism provided in the present disclosure has an enhanced activity and/or expression level of the lysine efflux protein, and can efflux lysine out of the cells of the microorganism efficiently. The recombinant microorganism of the present disclosure has a significantly improved lysine yield and sugar-acid conversion rate, which is suitable for large-scale industrial production of lysine and has important prospect in industrial application.

In some embodiments, the construction method for the recombinant microorganism provided in the present disclosure enables a recombinant microorganism having a high lysine yield and a high sugar-acid conversion rate by enhancing the protein activity and/or protein expression level of the lysine efflux protein. Moreover, the method is simple in preparation process, easy to implement, and suitable for large-scale production of recombinant microorganisms.

In some embodiments, the method for producing the lysine provided in the present disclosure allows for fermentation production of lysine using a recombinant microorganism, has the advantages of a high sugar-acid conversion rate and a high lysine yield, and a simplified process and reduced costs, and is suitable for large-scale industrial production of lysine.

### BRIEF DESCRIPTION OF THE DRAWINGS

FIG. 1 shows the analysis results of the transmembrane domains of the lysine efflux proteins (EcLysO, BsLysO, HgLysO, FsLysO) of the LysO family, in which the bolded segments marked by arrows are transmembrane domains.
FIG. 2 shows the analysis results of the transmembrane domains of the lysine efflux proteins (MsLysO, KgLysO, PvLysO) of the LysO family, in which the bolded segments marked by arrows are transmembrane domains.

### DETAILED DESCRIPTION

### Definitions

When used in combination with the term "including" in the claims and/or specification, the word "a" or "an" may refer to "one", or refer to "one or more", "at least one", and "one or more than one".

As used in the claims and specification, the term "including", "having", "comprising", or "containing" is intended to be inclusive or open-ended, and does not exclude additional or unrecited elements or methods and steps.

Throughout the application document, the term "about" means that a value includes the standard deviation of the error caused by the device or method used to measure the value.

It is applicable to the content disclosed herein that the term "or" is defined only as alternatives and "and/or", but the term "or" used herein refers to "and/or" unless otherwise expressly stated to be only alternatives or mutual exclusion between alternatives.

The selected/optional/preferred "numerical range", when used in the claims or the specification, includes not only the numerical endpoints at both ends of the range, but also all natural numbers covered between the above numerical endpoints with respect to these numerical endpoints.

The terms "polypeptide", "peptide", and "protein" are used interchangeably herein and are a polymer of amino acids of any length. The polymer may be linear or branched. It may comprise modified amino acids, and may be interrupted by non-amino acids. The term also includes amino acid polymers that have been modified (e.g., disulfide bond formation, glycosylation, lipidation, acetylation, phosphorylation, or any other manipulations, such as conjugation with a labeling component).

The term "amino acid efflux protein" as used herein refers to a protein capable of exporting intracellular amino acids out of cells. The cells transport amino acids mainly by the exporters on cell membranes, which can be divided into different families according to the structure, transport mode, energy coupling mechanism, and functional characteristics of the exporters. Among them, the proteins responsible for efflux of amino acids in amino acid exporters are amino acid efflux proteins. In some embodiments, the amino acid efflux proteins of the present disclosure are lysine efflux proteins.

As used herein, the term "wild-type" refers to an object that can be found in nature. For example, a polypeptide or a polynucleotide sequence present in an organism that can be isolated from one source in nature and has not been intentionally modified by human in the laboratory is naturally occurring.

As used herein, the term "microorganism" is a collective term for tiny organisms that are difficult to observe with naked eyes, including bacteria, fungi and so forth. Because of their large ratio of surface area to volume, microorganisms can quickly exchange substances with the external environment and produce metabolites. The microorganism in the present disclosure refers particularly to a fermentative microorganism that can be cultured by fermentation to produce metabolites such as sugars, lipids, amino acids, and nucleotides.

The term "wild-type microorganism" used herein may be a microorganism that has not been artificially modified and can be found in nature. Besides, the wild-type microorganism also includes derivative microorganisms that have undergone some modification or transformation. It is to be noted that in the present disclosure, a microorganism may be considered as a wild-type microorganism as long as it is not subjected to any one of the following modifications: introduction of the MsLysO protein or enhancement of its expression level, introduction of a mutant in which the MsLysO protein activity is maintained or enhancement of its expression level, introduction of the PvLysO protein or enhancement of its expression level, and introduction of a mutant in which the PvLysO protein activity is maintained or enhancement of its expression level.

The term "recombinant microorganism" as used herein is a modified microorganism obtained by recombination by genetic engineering. The embodiment includes, but is not limited to, an increase in the copy number of the protein coding gene, a modification of the sequence of the protein coding gene, introduction of exogenous recombinant gene and so forth. The term "recombinant gene" is not a naturally existing gene. The recombinant gene includes a protein coding sequence operably linked to the expression regulatory sequence. The embodiment includes, but is not limited to, exogenous genes introducing the microorganism, endogenous protein coding sequences operably linked to a heterologous promoter and genes having a modified protein coding sequence. The recombinant genes are preserved on the genome of the microorganism, plasmids in the microorganism or bacteriophages in the microorganism.

As used herein, the term "coding gene" refers to a DNA molecule capable of guiding synthesis of a protein according to a certain rule. The process of a protein coding gene to guide synthesis of protein generally includes a transcription process using the double-stranded DNA as a template and a translation process using mRNA as a template. The coding gene contains a Coding Sequence (CDS sequence), and can guide production of mRNA encoding the protein. Exemplarily, the protein coding gene of the present disclosure is the gene encoding the amino acid efflux protein. In some embodiments, the protein coding gene of the present disclosure is the gene encoding a polypeptide having a sequence as set forth in SEQ ID NO: 4 or SEQ ID NO: 6 or a mutant in which the activity of the amino acid efflux protein is maintained.

In some embodiments, the recombinant microorganism having the enhanced protein activity, enhanced expression level of the protein coding gene, enhanced enzyme activity or enhanced expression level of enzyme coding gene includes recombinant microorganisms modified by the following genetic engineering methods: introduction of strong promoters or strong ribosome binding sites into cells of microorganisms, introduction of non-integral recombinant expression vectors of proteins, and introduction of chromosomally integrated recombinant expression vectors of proteins; alterations of the promoters, translation regulatory region or codon in the coding region of the coding gene to enhance its transcription or translation; alternation of the sequence of the coding gene so that its mRNA is steadily enhanced or the structure of the coding protein is stable; or any other methods for enhancing the activity by modifying the gene coding region and the adjacent upstream and downstream regions, etc.

In some embodiments, the recombinant microorganisms of the present disclosure are recombinant microorganisms obtained by modifying one or two or more of the following microorganisms by genetic engineering: microorganisms of *Escherichia, Erwinia, Serratia, Providencia, Enterobacteria, Salmonella, Streptomyces, Pseudomonas, Brevibacterium* or *Corynebacterium.*

The term "mutant" as used herein refers to a polynucleotide or polypeptide that, relative to the "wild-type" or "comparative" polynucleotide or polypeptide, contains alternation(s) (i.e., substitution, insertion and/or deletion) of a polynucleotide or polypeptide at one or more (e.g., several) positions, where the substitution refers to a substitution of a different nucleotide or amino acid for a nucleotide or amino acid that occupies one position; the deletion refers to removal of a nucleotide or amino acid that occupies certain position; and the insertion refers to an addition of a nucleotide or amino acid after the nucleotide or amino acid adjacent to and immediately following the occupied position.

In some embodiments, the "mutation" of the present disclosure may be selected from "conservative mutations". In the present disclosure, the term "conservative mutations" refers to mutations that can normally maintain the functions of the protein. A representative example of the conservative mutations is conservative substitutions.

As used herein, the term "conservative substitution" is usually to exchange one kind of amino acid at one or more sites of a protein. Such a substitution may be conservative. Substitutions taken as conservative substitutions may include, for example, a substitution of Ala with Ser or Thr, a substitution of Arg with Gln, His or Lys, a substitution of Asn with Glu, Gln, Lys, His or Asp, a substitution of Asp with Asn, Glu or Gln, a substitution of Cys with Ser or Ala, a substitution of Gin with Asn, Glu, Lys, His, Asp or Arg, a substitution of Glu with Gly, Asn, Gln, Lys or Asp, a substitution of Gly with Pro, a substitution of His with Asn, Lys, Gln, Arg or Tyr, a substitution of Ile with Leu, Met, Val or Phe, a substitution of Leu with Ile, Met, Val or Phe, a substitution of Lys with Asn, Glu, Gln, His or Arg, a substitution of Met with Ile, Leu, Val or Phe, a substitution of Phe with Trp, Tyr, Met, Ile or Leu, a substitution of Ser with Thr or Ala, a substitution of Thr with Ser or Ala, a substitution of Trp with Phe or Tyr, a substitution of Tyr with His, Phe or Trp, and a substitution of Val with Met, Ile or Leu. In addition, conservative mutations further include mutations that naturally occur as a result of the individual difference, differences in strains and species, etc.

The term "polynucleotide" as used herein refers to a polymer composed of nucleotides. Polynucleotide may be in the form of an individual fragment, or may be a constituent part of a larger nucleotide sequence structure, which is derived from a nucleotide sequence that has been isolated at least once in quantity or concentration, and could be recognized, manipulated, and recovered from the sequence and its component nucleotide sequence by a standard molecular biological method (*e.g.,* using a cloning vector). When a nucleotide sequence is represented by a DNA sequence (*i.e.,* A, T, G, C), it also includes an RNA sequence (*i.e.,* A, U, G, C), where "U" substitutes for "T". In other words, "polynucleotide" refers to a nucleotide polymer removed from other nucleotides (an individual fragment or an entire fragment), or may be a constituent part or component of a larger nucleotide structure, such as an expression vector or a polycistronic sequence. Polynucleotides include DNA, RNA, and cDNA sequences.

As used herein, the terms "sequence identity" and "percent identity" refer to the percentage of nucleotides or amino acids that are the same (*i.e.,* identical) between two or more polynucleotides or polypeptides. The sequence identity between two or more polynucleotides or polypeptides may be determined by aligning the nucleotide sequences of polynucleotides or the amino acid sequences of polypeptides and scoring the number of positions at which nucleotide or amino acid residues are identical in the aligned polynucleotides or polypeptides, and comparing the number of these positions with the number of positions at which nucleotide or amino acid residues are different in the aligned polynucleotides or polypeptides. Polynucleotides may differ at one position by, *e.g.,* containing a different nucleotide (*i.e.,* substitution or mutation) or deleting a nucleotide (*i.e.,* insertion or deletion of a nucleotide in one or two polynucleotides). Polypeptides may differ at one position by, *e.g.,* containing a different amino acid (*i.e.,* substitution or mutation) or deleting an amino acid (*i.e.,* insertion or deletion of an amino acid in one or two polypeptides). The sequence identity may be calculated by dividing the number of positions at which nucleotide or amino acid residues are identical by the total number of nucleotide or amino acid residues in the polynucleotides or polypeptides. For example, the percent identity may be calculated by dividing the number of positions at which nucleotide or amino acid residues are identical by the total number of nucleotide or amino acid residues in the polynucleotides or polypeptides, and multiplying the result by 100.

In some embodiments, two or more sequences or subsequences, when compared and aligned at maximum correspondence by the sequence alignment algorithm or by the visual inspection measurement, have at least 80%, 81%, 82%, 83%, 84%, 85%, 86%, 87%, 88%, 89%, 90%, 91%, 92%, 93%, 94%, 95%, 96%, 97%, 98%, or 99% "sequence identity" or "percent identity" of nucleotides. In some embodiments, the sequence is substantially identical over the full length of either or both of the compared biopolymers (*e.g.,* polynucleotides).

As used herein, the term "enzyme activity" or "protein activity" may also be expressed as "specific activity", which have the same meaning in the present disclosure and may be used interchangeably. It refers to the enzyme activity (U/mg) or protein activity (U/mg) per gram of polypeptide.

The term "expression" as used herein includes any steps involving the RNA production and protein production, which include, but are not limited to, transcription, post-transcriptional modification, translation, post-translational modification, and secretion.

As used herein, the term "vector" refers to a DNA construct containing DNA sequences operably linked to appropriate control sequences, so as to express a gene of interest in a suitable host. "Recombinant expression vector" refers to a DNA structure used to express, for example, a polynucleotide encoding a desired polypeptide. The recombinant expression vector may include, for example: i) a collection of genetic elements having a regulatory effect on gene expression, such as promoters and enhancers; ii) a structure or coding sequence that is transcribed into mRNA and translated into a protein; and iii) transcriptional subunits that appropriately transcribe and translate initiation and termination sequences. The recombinant expression vectors are constructed in any suitable manner. The nature of the vector is not critical, and any vector may be used, including plasmids, viruses, phages, and transposons. Potential vectors used herein include, but are not limited to, chromosomal, non-chromosomal, and synthetic DNA sequences, such as bacterial plasmids, phage DNAs, yeast plasmids, and vectors derived from combinations of plasmids and phage DNAs, and DNAs from viruses such as vaccinia, adenovirus, avian pox, baculovirus, SV40, and pseudorabies. The terms "recombinant expression vector" and "recombinant vector" may be used herein interchangeably.

The term "host cell" as used herein is intended to mean any cell type that is easily transformed, transfected, transduced or the like of the nucleic acid construct or the expression vector of the polynucleotide of the present disclosure. The term "recombinant host cell" encompasses host cells that are different from the parent cell after the introduction of a polynucleotide encoding the lysine efflux protein, a nucleic acid construct or a recombinant expression vector. The recombinant host cell is specifically achieved by transformation.

As used herein, the term "lysine producing strain" is intended to mean any type of strain usable for production of lysine. The source of the strain includes, but is not limited to, *Escherichia, Erwinia, Serratia, Providencia, Enterobacteria, Salmonella, Streptomyces, Pseudomonas, Brevibacterium* or *Corynebacterium.* In some embodiments, the lysine producing strain is derived from *Corynebacterium glutamicum* or *Escherichia coli.* In some preferred embodiments, the lysine producing strain is derived from *Corynebacterium glutamicum* ATCC 13032, *Corynebacterium glutamicum* ATCC 13869, *Corynebacterium glutamicum* ATCC 14067 or a derivative strain of any one thereof.

In some embodiments, the lysine producing strains may be strains expressing the aspartate kinase LysC that relieves the feedback inhibition based on *Corynebacterium glutamicum* ATCC 13032. The LysC that relieves the feedback inhibition, such as LysC^{T311I}, LysC^{S301F}, LysC^{L301K}, LysC^{L301M}, LysC^{FBR}, LysC^{I293Y}, LysC^{I293Q}, LysC^{D294F} or LysC^{T307G}, has been reported in the prior art. In addition, the lysine producing strains may also be other species of strains capable of producing lysine.

In some embodiments, the lysine producing strains may further include, but not limited to, one or more genes selected from the following genes that are attenuated or reduced in expression:
a. *adhE* gene encoding ethanol dehydrogenase;
b. *ackA* gene encoding acetate kinase;
c. *pta* gene encoding phosphate acetyltransferase;
d. *IdhA* gene encoding lactic dehydrogenase;
e. *focA* gene encoding formate transporter;
f. *pflB* gene encoding pyruvate formate lyase;
g. *poxB* gene encoding pyruvate oxidase;
h. *thrA* gene encoding aspartate kinase I/homoserine dehydrogenase I as a bifunctional enzyme;
i. *thrB* gene encoding homoserine kinase;
j. *ldcC* gene encoding lysine decarboxylase; and
h. *cadA* gene encoding lysine decarboxylase.

In some embodiments, the lysine producing strains may further include, but not limited to, one or more genes selected from the following genes that are enhanced or overexpressed:
a. *dapA* gene encoding dihydrodipyridine synthase that relieves feedback inhibition of lysine;
b. *dapB* gene encoding dihydrodipicolinate reductase;
c. *ddh* gene encoding diaminopimelate dehydrogenase;
d. *dapD* encoding tetrahydrodipicolinate succinylase and *dapE* encoding succinyldiaminopimelate deacylase;
e. *asd* gene encoding aspartate-semialdehyde dehydrogenase;
f. *ppc* gene encoding phosphoenolpyruvate carboxylase;
g. *pntAB* gene encoding niacinamide adenine dinucleotide transhydrogenase; and
i. *lysE* gene encoding transporter of lysine.

The term "transformation" used herein has the meaning generally understood by a person skilled in the art, namely, a process of introducing an exogenous DNA into a host. Methods for the transformation include any method for introducing a nucleic acid into a cell. These methods include, but are not limited to, electroporation, calcium phosphate precipitation, calcium chloride precipitation (CaCl₂), microinjection, a polyethylene glycol (PEG) method, a DEAE-dextran method, a cationic liposome method, and a lithium acetate-DMSO method.

As used herein, the term "amino acid" or "L-amino acid" refers to a basic constituent unit of a protein in which the amino group and the carboxyl group bind to the same carbon atom. Exemplarily, the amino acid is one or more selected from the group consisting of: glycine, alanine, valine, leucine, isoleucine, threonine, serine, cysteine, glutamine, methionine, aspartic acid, asparagine, glutamic acid, lysine, arginine, histidine, phenylalanine, tyrosine, tryptophan, proline, hydroxyproline, 5-aminolevulinic acid or a derivative of any one of the above amino acids. In addition, the amino acid may also be other types of amino acids in the art. In specific embodiments of the present disclosure, the amino acid is lysine.

The term "culture" used herein may be the culture carried out by conventional methods in the art, which includes, but is not limited to, well-plate culture, shaking culture, batch culture, continuous culture, fed-batch culture, etc. Also, various culture conditions such as the temperature, time, and pH value of the medium may be properly adjusted by actual situations.

Unless otherwise defined in the invention or clearly indicated by context, all technical and scientific terms used herein have the same meanings as typically understood by one of ordinary skill in the art to which the present disclosure belongs.

### Lysine Efflux Protein

*Escherichia* coli-derived LysO family proteins can efflux L-lysine efficiently and significantly improve the lysine yield, and their abilities to efflux L-arginine and to increase the L-arginine yield are remarkably lower as compared to L-lysine. When protein sequences are searched in the NCBI database taking "lysine exporter LysO" as keywords, there are lysine efflux proteins of the LysO family derived from more than 27,000 strains. Nevertheless, the *Escherichia coli-*derived LysO is the sole identified LysO family protein at present. There is an urgent need in this field to discover more lysine efflux proteins efficient in efflux of lysine.

The number of the transmembrane domain is crucial to the functions of the exporter. The *Escherichia coli*-derived LysOs (EcLysOs) that have currently been found have 8 transmembrane domains. The present disclosure has found that an LysO family lysine efflux protein having 9 transmembrane domains may have the corresponding functions, and eventually found that the *unclassified Marinobacter-derived* MsLysO (NCBI Accession No.: WP_123634637.1, SEQ ID NO: 4) and *Pseudomonas viridiflava-derived* PvLysO (NCBI Accession No.: WP_122810318.1, SEQ ID NO: 6) have a significant lysine efflux activity, which provides a novel lysine efflux protein for modifications of lysine high-producing strains.

In some embodiments, the lysine efflux proteins MsLysO and PvLysO of the present disclosure efflux lysine with higher substrate specificity, and may serve as lysine efflux proteins to realize efficient efflux of lysine, so as to increase the lysine yield.

In some embodiments, the protein activities of MsLysO and PvLysO to efficiently efflux lysine may also be made use of to prepare products that efflux lysine out of the cells of microorganisms to improve the efficiency in transfer of intracellular lysine out of the cells.

In some embodiments, the present disclosure provides a MsLysO mutant, which is a mutant having one or more amino acids substituted, repeated, deleted or added at one or more positions of the sequence set forth in SEQ ID NO: 4 and with the MsLysO protein activity maintained. Exemplarily, the MsLysO mutant is a mutant having at least 80%, 81%, 82%, 83%, 84%, 85%, 86%, 87%, 88%, 89%, 90%, 91%, 92%, 93%, 94%, 95%, 96%, 97%, 98%, or 99% (including all ranges and percentages between these numerical values) sequence identity to MsLysO. The MsLysO mutant may be used as a lysine efflux protein to improve the capability of microorganisms to efflux lysine.

In some embodiments, the present disclosure provides a PvLysO mutant, which is a mutant having one or more amino acids substituted, repeated, deleted or added at one or more positions of the sequence set forth in SEQ ID NO: 6 and with the activity of the lysine efflux protein maintained. Exemplarily, the PvLysO mutant is a mutant having at least 80%, 81%, 82%, 83%, 84%, 85%, 86%, 87%, 88%, 89%, 90%, 91%, 92%, 93%, 94%, 95%, 96%, 97%, 98%, or 99% (including all ranges and percentages between these numerical values) sequence identity to PvLysO. The PvLysO mutant may be used as a lysine efflux protein to improve the capability of microorganisms to efflux lysine.

In some embodiments, the present disclosure provides a polynucleotide encoding MsLysO. The polynucleotide encoding MsLysO is further operably linked to the regulatory sequence and used to construct a nucleic acid construct, a recombinant expression vector, a recombinant host cell and so forth that express the MsLysO protein. By expressing the MsLysO protein having the lysine efflux activity, the above polynucleotide, nucleic acid construct, recombinant expression vector, recombinant host cell and so forth may facilitate efficient efflux of lysine out of the cells, thereby improving the substrate conversion rate and the lysine yield.

In some specific embodiments, the polynucleotide encoding MsLysO includes the nucleotide sequence as set forth in SEQ ID NO: 10, or nucleotide sequences having at least 80%, 81%, 82%, 83%, 84%, 85%, 86%, 87%, 88%, 89%, 90%, 91%, 92%, 93%, 94%, 95%, 96%, 97%, 98%, or 99% (including all ranges and percentages between these numerical values) sequence identity to the sequence as set forth in SEQ ID NO: 10 and encoding polypeptides having the MsLysO protein activity.

In some embodiments, the present disclosure provides a polynucleotide encoding PvLysO. The polynucleotide encoding PvLysO is further operably linked to the regulatory sequence and used to construct a nucleic acid construct, a recombinant expression vector, a recombinant host cell and so forth that express the PvLysO protein. By expressing the PvLysO protein having the lysine efflux activity, the above polynucleotide, nucleic acid construct, recombinant expression vector, recombinant host cell and so forth may facilitate efficient efflux of lysine out of the cells, thereby improving the substrate conversion rate and the lysine yield.

In some specific embodiments, the polynucleotide encoding PvLysO includes the nucleotide sequence as set forth in SEQ ID NO: 12, or nucleotide sequences having at least 80%, 81%, 82%, 83%, 84%, 85%, 86%, 87%, 88%, 89%, 90%, 91%, 92%, 93%, 94%, 95%, 96%, 97%, 98%, or 99% (including all ranges and percentages between these numerical values) sequence identity to the sequence as set forth in SEQ ID NO: 12 and encoding polypeptides having the PvLysO protein activity.

### Lysine Producing Strain

The lysine producing strain of the present disclosure is a recombinant microorganism obtained by modifying the wild-type microorganism so that it has an enhanced activity and/or expression level of the lysine efflux protein.

In some embodiments, the lysine efflux protein is one or a combination of two or more selected from the group consisting of: MsLysO, a mutant having the MsLysO protein activity maintained, PvLysO, and a mutant having the PvLysO protein activity maintained.

In some specific embodiments, the present disclosure constructs a recombinant microorganism by enhancing the protein activity and/or protein expression level of MsLysO or a mutant thereof, for example, increasing the copy number of the coding gene of the MsLysO protein, modifying the sequence of the coding gene of the MsLysO protein, introducing exogenous genes by means of plasmid episomal expression or chromosomal integration, or introducing strong promoters, etc.

In some more specific embodiments, the present disclosure clones the protein-coding gene *MsLysO* that encodes MsLysO into the downstream of the promoter of the pEC-*ccdB* plasmid to construct a recombinant expression vector pEC-*MsLysO* that expresses MsLysO. The recombinant expression vector pEC-*MsLysO* is transformed into a host cell to obtain a lysine producing strain. In other embodiments, the *MsLysO* gene may also be used to construct other kinds of recombinant expression vectors, and lysine producing strains are then constructed using the desired kinds of recombinant expression vectors.

In some specific embodiments, the present disclosure constructs a recombinant microorganism by enhancing the protein activity and/or protein expression level of PvLysO or a mutant thereof, for example, increasing the copy number of the coding gene of the PvLysO protein, modifying the sequence of the coding gene of the PvLysO protein, introducing exogenous genes by means of plasmid episomal expression or chromosomal integration, or introducing strong promoters, etc.

In some more specific embodiments, the present disclosure clones the protein-coding gene *PvLysO* that encodes PvLysO into the downstream of the promoter of the pEC-*ccdB* plasmid to construct a recombinant expression vector pEC-*PvLysO* that expresses PvLysO. The recombinant expression vector *pEC-PvLysO* is transformed into a host cell to obtain a lysine producing strain. In other embodiments, the *PvLysO* gene may also be used to construct other kinds of recombinant expression vectors, and lysine producing strains are then constructed using the desired kinds of recombinant expression vectors.

In some embodiments, the microorganism for constructing a lysine producing strain in the present disclosure includes, but is not limited to, microorganisms derived from *Escherichia, Erwinia, Serratia, Providencia, Enterobacteria, Salmonella, Streptomyces, Pseudomonas, Brevibacterium* or *Corynebacterium.*

In some specific embodiments, the lysine producing strain is derived from *Corynebacterium glutamicum* or *Escherichia coli.*

In some preferred embodiments, the lysine producing strain is derived from *Corynebacterium glutamicum* ATCC 13032, *Corynebacterium glutamicum* ATCC 13869, *Corynebacterium glutamicum* ATCC 14067 or a derivative strain of any one thereof.

In some specific embodiments, the lysine producing strain is constructed based on a derivative strain of *Corynebacterium glutamicum* ATCC 13032. Exemplarily, the derivative strain of *Corynebacterium glutamicum* ATCC 13032 is the L-lysine producing strain SCgL42.

In other embodiments, the host cell for constructing the lysine producing strain may further include, but not limited to, one or more genes selected from the following genes that are attenuated or reduced in expression:
a. *adhE* gene encoding ethanol dehydrogenase;
b. *ackA* gene encoding acetate kinase;
c. *pta* gene encoding phosphate acetyltransferase;
d. *ldhA* gene encoding lactic dehydrogenase;
e. *focA* gene encoding formate transporter;
f. *pflB* gene encoding pyruvate formate lyase;
g. *poxB* gene encoding pyruvate oxidase;
h. *thrA* gene encoding aspartate kinase I/homoserine dehydrogenase I as a bifunctional enzyme;
i. *thrB* gene encoding homoserine kinase;
j. *ldcC* gene encoding lysine decarboxylase; and
h. *cadA* gene encoding lysine decarboxylase.

In some embodiments, the host cell for constructing the lysine producing strain may further include, but not limited to, one or more genes selected from the following genes that are enhanced or overexpressed:
a. *dapA* gene encoding dihydrodipyridine synthase that relieves feedback inhibition of lysine;
b. *dapB* gene encoding dihydrodipicolinate reductase;
c. *ddh* gene encoding diaminopimelate dehydrogenase;
d. *dapD* encoding tetrahydrodipicolinate succinylase and *dapE* encoding succinyldiaminopimelate deacylase;
e. *asd* gene encoding aspartate-semialdehyde dehydrogenase;
f. *ppc* gene encoding phosphoenolpyruvate carboxylase;
g. *pntAB* gene encoding niacinamide adenine dinucleotide transhydrogenase; and
i. *lysE* gene encoding transporter of lysine.

In some embodiments, the lysine producing strains constructed in the present disclosure are effective in promoting transfer of lysine out of the cells due to the enhanced activities and/or protein expression levels of their lysine efflux proteins, thereby improving the sugar-acid conversion rate and lysine yield of the lysine producing strains.

In some specific embodiments, the sugar-acid conversion rate of the lysine producing strain is improved by at least 10%, at least 20%, at least 30%, at least 40%, at least 50%, at least 60%, at least 70%, at least 80%, at least 90%, at least 1 fold, at least 1.5 folds, at least 2 folds, at least 2.5 folds, at least 3 folds or the like as compared to the wild-type strain with an unmodified lysine efflux protein.

In some specific embodiments, the lysine yield of the lysine producing strain is improved by at least 10%, at least 20%, at least 30%, at least 40%, at least 50%, at least 60%, at least 70%, at least 80%, at least 90%, at least 1 fold, at least 1.5 folds, at least 2 folds, at least 2.5 folds, at least 3 folds or the like as compared to the wild-type strain with an unmodified lysine efflux protein.

In some embodiments, a further gene modification of the lysine producing strain may further improve the lysine yield of the lysine producing strain. Exemplarily, the gluconate repressor GntR1 in the lysine producing strain is mutated, such that arginine at position 102 of the gluconate repressor GntR1 is mutated to alanine to obtain a lysine producing strain with a further improved lysine yield.

In some specific embodiments, by further introducing an R102A mutation of the gluconate repressor GntR1 to the SCgL42 (pEC-*MsLysO*) recombinant strain in which pEC-*MsLysO* has been transformed, it has been found that the mutant strain has a further improved lysine yield.

### Production Method for Lysine

The method for producing the lysine provided in the present disclosure may comprise the following steps:
(1) subjecting a lysine producing strain to fermentation culture; and
(2) collecting the fermentation reaction solution at the end of the fermentation reaction, and isolating the lysine therefrom.

The lysine producing strain of the present disclosure has an enhanced activity and/or expression level of the lysine efflux protein, significantly improved capacity of transferring lysine out of cells, and a high amino acid yield and sugar-acid conversion rate, and is suitable for large-scale industrial production of lysine.

In some embodiments, the lysine producing strain is subjected to seed culture and then the culture is inoculated in a fermentation medium for fermentation culture.

Exemplarily, the strains are firstly inoculated into a TSB liquid medium and cultured for 8 h. The cultures are inoculated as seeds into a 24-well plate containing 800 µl of fermentation media in each well with an initial OD₆₀₀ controlled to about 0.1, and cultured at 30°C for 19 h. The rotating speed of the plate shaker is 800 rpm. Three samples are set for each strain. After fermentation, the L-lysine yield and glucose consumption are detected.

In some specific embodiments, the ingredients (g/L) of the TSB seed medium are as follows: glucose, 5 g/L; yeast powder, 5 g/L; soy peptone, 9 g/L; urea, 3 g/L; succinic acid, 0.5 g/L; K₂HPO₄·3H₂O, 1 g/L; MgSO₄·7H₂O, 0.1 g/L; biotin, 0.01 mg/L; vitamin B1, 0.1 mg/L; and MOPS, 20 g/L.

In some specific embodiments, the ingredients of the fermentation medium are as follows: glucose, 80 g/L; yeast powder, 1 g/L; soy peptone, 1 g/L; NaCl, 1 g/L; ammonium sulfate, 1 g/L; urea, 8 g/L; K₂HPO₄·3H₂O, 1 g/L; MgSO₄·7H₂O, 0.45 g/L; FeSO4·7H₂O, 0.05 g/L; biotin, 0.4 mg/L; vitamin B1, 0.1 mg/L; MOPS, 40 g/L; and initial pH7.2.

In some specific embodiments, 25 mg/L of kanamycin is added in both the seed culture and the fermentation process.

In some embodiments, the method for isolating lysine may be a method commonly used in this field, including but not limited to: filtration, anion-exchange chromatography, crystallization or HPLC.

In this field, the methods for manipulating microorganisms are known, and are described in publications such as Modern Methods in Molecular Biology (Online ISBN: 9780471142720, John Wiley and Sons, Inc.), Microbial Metabolic Engineering: Methods and Protocols (Qiong Cheng Ed., Springer) and Systems Metabolic Engineering: Methods and Protocols (Hal S. Alper Ed., Springer).

### Examples

Other objectives, features, and advantages of the present disclosure become apparent from the following detailed description. It should be appreciated, however, that the detailed description and specific examples (while indicating the specific embodiments of the present disclosure) are merely provided for illustrative purposes because various alterations and modifications made within the spirits and scope of the present disclosure will become apparent to a person skilled in the art after reading the detailed description.

The experimental techniques and experimental methods used in the examples, unless otherwise specified, are all conventional techniques and methods. For example, experimental methods for which no specific conditions are indicated in the following examples are generally performed according to conventional conditions such as those described by Sambrook et al., Molecular Cloning: A Laboratory Manual (New York: Cold Spring Harbor Laboratory Press, 1989), or those recommended by manufacturers. The materials, reagents, etc. used in the examples, unless otherwise specified, are all commercially available.

### Example 1: Genome mining of novel L-lysine efflux protein

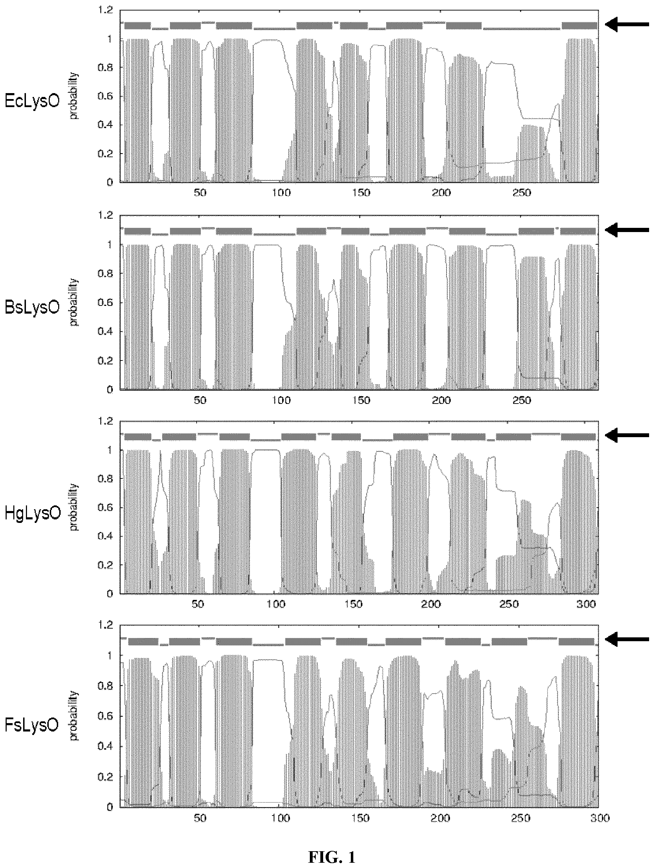

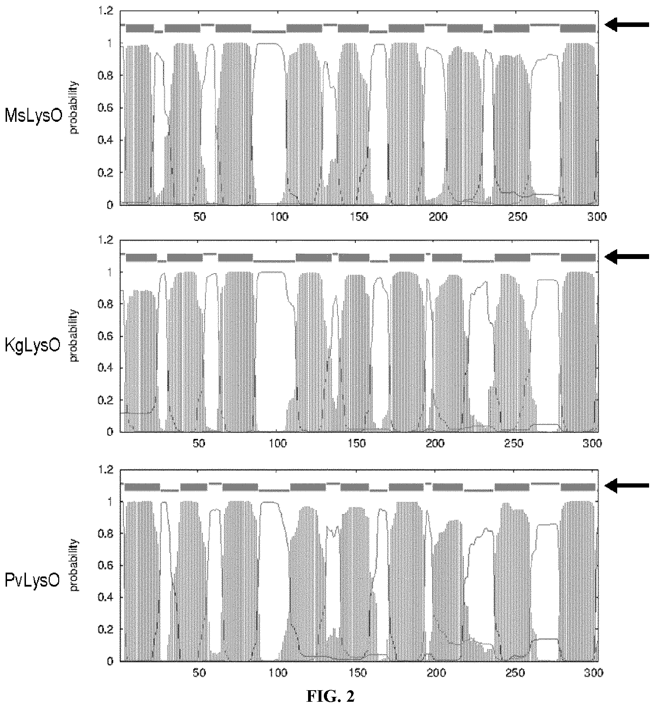

After analysis of the structure of the *Escherichia coli-*derived LysO (EcLysO, also referred to as YbjE) protein, it was found that this protein had 8 transmembrane domains (FIG. 1). Since the number of the transmembrane domains was crucial to the functions of the exporters, we analyzed the structures of the currently available polypeptides annotated as lysine efflux proteins and found LysO family lysine efflux proteins having 9 transmembrane domains. These proteins might have, by preliminary conjecture, a better capacity to efflux lysine. Considering the species origin and evolutionary genetic relationship, we finally selected six different kinds of LysO family lysine efflux proteins having 9 transmembrane domains (FIGS. 1 and 2), which were *Brenneria salicis*-derived BsLysO (NCBI Accession No.: WP_113868262.1, SEQ ID NO: 1), *Halomonas gudaonensis*-derived HgLysO (NCBI Accession No.: WP 089685822.1, SEQ ID NO: 2), *Ferrimonas sediminum*-derived FsLysO(NCBI Accession No.: WP_090365555.1, SEQ ID NO: 3), *unclassified Marinobacter*-derived MsLysO (NCBI Accession No.: WP_123634637.1, SEQ ID NO: 4), *Kerstersia gyiorum*-derived KgLysO (NCBI Accession No.: WP_068372414.1, SEQ ID NO: 5), and *Pseudomonas viridiflava-*derived PvLysO (NCBI Accession No.: WP_122810318.1, SEQ ID NO: 6), respectively.

### Example 2: Construction of strains expressing novel L-lysine efflux proteins

The expression plasmids were constructed as follows: The selected lysine efflux proteins were expressed on the *pEC-ccdB* plasmid - a derivative plasmid of the pEC-XK99E plasmid (GenBank: AY219683.1). The pEC-*ccdB* plasmid was enzyme digested with *Bsa* I, and the selected efflux protein genes were cloned into the downstream of the promoter of the plasmid. The same expression element was used to express the lysine efflux protein genes of different sources. The lysine efflux protein genes *BsLysO* (SEQ ID NO: 7), *HgLysO* (SEQ ID NO: 8), *FsLysO* (SEQ ID NO: 9), *MsLysO* (SEQ ID NO: 10), *KgLysO* (SEQ ID NO: 11), and *PvLysO* (SEQ ID NO: 12) were sent to GENEWIZ for the whole gene synthesis respectively, and cloned into the *pEC-ccdB* plasmid to obtain pEC-*BsLysO,* pEC-*HgLysO,* pEC-*FsLysO,* pEC-*MsLysO, pEC-KgLysO,* and pEC-*PvLysO* plasmids, respectively. At the same time, the known lysine efflux protein genes - *Corynebacterium glutamicum-derived LysE* gene (Gene No. Cgl1262 in *Corynebacterium glutamicum* ATCC 13032 genome GenBank: BA000036.3 in NCBI, NCBI-Protein ID: BAB98655) and *Escherichia* coli-derived *LysO* gene (NCBI-Gene ID: 947142, NCBI-Protein ID: NP_415395) were cloned into the pEC-*ccdB* plasmid in the same way as described above to obtain two control plasmids pEC-*CgLysE* and pEC-*EcLysO;* and a control plasmid pEC-control was constructed without any gene cloned thereon.

In order to evaluate the effects of the lysine efflux proteins on efflux of lysine in *Corynebacterium glutamicum,* the constructed pEC-*CgLysE,* pEC-*EcLysO,* pEC-*BsLysO,* pEC-*HgLysO,* pEC-*FsLysO,* pEC-*MsLysO,* pEC-*KgLysO,* pEC-*PvLysO,* and pEC-control plasmids were transformed into a *Corynebacterium glutamicum* L-lysine producing strain SCgL42 (referring to the patent CN112695036A, which was incorporated herein by reference) respectively to obtain efflux protein expression strains and control strains: SCgL42 (pEC-*CgLysE*), SCgL42 (pEC-*EcLysO*), SCgL42 (pEC-*BsLysO*), SCgL42 (pEC-*HgLysO*), SCgL42 (pEC*-FsLysO*), SCgL42 *(pEC-MsLysO),* SCgL42 (pEC-*KgLysO*), SCgL42 *(pEC-PvLysO),* and SCgL42 (pEC-control).

### Example 3: Evaluation on novel lysine efflux proteins in intensifying efflux of lysine to increase lysine yield

Expressing lysine efflux proteins could intensify the capacity of strains to efflux lysine, thereby increasing the yield of extracellular lysine. Patents such as CN102947460A all confirmed whether the efflux proteins could enhance efflux of amino acids to improve the yield depending upon whether the expression of efflux proteins increased the yield of extracellular amino acids. The present disclosure evaluated the effects of expression of the lysine efflux proteins in *Corynebacterium glutamicum* on extracellular production of L-lysine by strains to thereby evaluate whether the lysine efflux proteins could promote lysine efflux from *Corynebacterium glutamicum.* SCgL42 (pEC-*CgLysE*), SCgL42 (pEC-*EcLysO*), SCgL42 (pEC-*BsLysO*), SCgL42 (pEC-*HgLysO*), SCgL42 (pEC-*FsLysO*), SCgL42 (pEC*-MsLysO*), SCgL42 (pEC-*KgLysO*), SCgL42 *(pEC-PvLysO),* and SCgL42 (pEC-control) strains were subjected to the lysine fermentation test, respectively.

The ingredients (g/L) of the TSB seed medium were as follows: glucose, 5 g/L; yeast powder, 5 g/L; soy peptone, 9 g/L; urea, 3 g/L; succinic acid, 0.5 g/L; K₂HPO₄·3H₂O, 1 g/L; MgSO₄·7H₂O, 0.1 g/L; biotin, 0.01 mg/L; vitamin B1, 0.1 mg/L; and MOPS, 20 g/L.

The ingredients of the fermentation medium were as follows: glucose, 80 g/L; yeast powder, 1 g/L; soy peptone, 1 g/L; NaCl, 1 g/L; ammonium sulfate, 1 g/L; urea, 8 g/L; K₂HPO₄·3H₂O, 1 g/L; MgSO₄·7H₂O, 0.45 g/L; FeSO4·7H₂O, 0.05 g/L; biotin, 0.4 mg/L; vitamin B1, 0.1 mg/L ; MOPS, 40 g/L; and initial pH7.2.

25 mg/L of Kanamycin was added to both the seed culture and the fermentation process. The strains were firstly inoculated into a TSB liquid medium and cultured for 8 h. The cultures were inoculated as seeds in a 24-well plate containing 800 µl of fermentation media in each well with the initial OD₆₀₀ controlled to about 0.1, and cultured at 30°C for 19 h. The rotating speed of the plate shaker was 800 rpm. Three samples were set for each strain. At the end of the fermentation, the L-lysine yield and the glucose consumption were detected. The sugar-acid conversion rates from glucose to L-lysine were detected by a biosensor SBA and calculated. The results were as shown in Table 1. Compared with the SCgL42 (pEC-control) control strain, the expression of both L-lysine efflux proteins MsLysO and PvLysO could improve the lysine yield by 115% and 137%, respectively; and could also improve the sugar-acid conversion rates from glucose to L-lysine by 117% and 153%, respectively. Compared with the known lysine efflux proteins CgLysE and EcLysO, the L-lysine efflux proteins MsLysO and PvLysO disclosed in the present disclosure further exhibited higher lysine yields and sugar-acid conversion rates from glucose to L-lysine. The expression of the lysine efflux protein KgLysO led to significant reductions in the lysine yield and sugar-acid conversion rate, which might be more conducive to efflux of other amino acids or metabolites, while the other three lysine efflux proteins did not show remarkable influences on the lysine yield and the sugar-acid conversion rate, which might not function in *Corynebacterium glutamicum.*

**Table 1 Effects of Expression of Lysine Efflux Proteins on Lysine Production**

| Strains | OD₆₀₀ | Lysine Yield (g/L) | Sugar-Acid Conversion Rate (%) |
|---|---|---|---|
| SCgL42 (pEC-control) | 18.8±0.7 | 3.6±0.0 | 6.3±0.0% |
| SCgL42 (pEC-*CgLysE*) | 15.8±1.0 | 5.6±0.4 | 9.2±0.8% |
| SCgL42 (pEC*-EcLysO*) | 17.3±1.0 | 6.4±0.4 | 10.7±0.9% |
| SCgL42 (pEC-*BsLysO*) | 19.4±1.8 | 3.5±0.2 | 6.3±0.7% |
| SCgL42 (pEC*-HgLysO*) | 18.2±1.2 | 3.3±0.2 | 6.2±0.4% |
| SCgL42 (pEC*-FsLysO*) | 16.5±1.4 | 3.1±0.5 | 5.7±1.2% |
| SCgL42 (pEC-*MsLysO*) | 19.5±0.9 | 7.7±0.5 | 13.6±0.6% |
| SCgL42 (pEC-*KgLysO*) | 18.1±2.3 | 1.1±0.2 | 2.0±0.5% |
| SCgL42 (pEC-*PvLysO*) | 17.6±1.2 | 8.5±0.2 | 15.9±0.9% |

Additionally, in order to further verify whether MsLysO and PvLysO exhibited the effect of effluxing lysine in different lysine producing strains to improve the lysine yield by strains, we transformed the plasmid pEC-*MsLysO* and the plasmid *pEC-PvLysO* constructed in Example 2 into a series of different lysine producing strains, including *Corynebacterium glutamicum* B253 (GeneBank accession number CP010451), *Corynebacterium glutamicum* SCgL30 (referring to CN112877269A, which was incorporated herein by reference), *Corynebacterium glutamicum* SCgL31 (referring to CN112877269A, which was incorporated herein by reference), *Corynebacterium glutamicum* SCgL40 (referring to CN113201536A, which was incorporated herein by reference), *Corynebacterium glutamicum* ZCgLJ6 (referring to CN113201514A, which was incorporated herein by reference), *Corynebacterium glutamicum* SCgL52 (referring to CN113249347A, which was incorporated herein by reference), and *Corynebacterium glutamicum* AHP and AHP (ΔCgl2639) (referring to CN113462583A, which was incorporated herein by reference), and obtained a series of recombinant strains after transfection: B253 (pEC-*MsLysO*), B253 (pEC-*PvLysO*), SCgL30 *(pEC-MsLysO),* SCgL30 *(pEC-PvLysO),* SCgL31 *(pEC-MsLysO),* SCgL31 (pEC-*PvLysO*), SCgL40 *(pEC-MsLysO),* SCgL40 *(pEC-PvLysO),* ZCgLJ6 *(pEC-MsLysO),* ZCgLJ6 *(pEC-PvLysO),* SCgL52 *(pEC-MsLysO),* SCgL52 *(pEC-PvLysO),* AHP *(pEC-MsLysO),* AHP *(pEC-PvLysO),* AHP (*ΔCgl2639,* pEC-*MsLysO*), and AHP (*ΔCgl2639,* pEC-*PvLysO*). The lysine fermentation test on the recombinant strains and the detection of lysine were carried out by the same method as described above. The results showed that overexpression of MsLysO and PvLysO in different starting strains could always improve the lysine yield. The above results suggested that the L-lysine efflux proteins MsLysO and PvLysO disclosed in the present disclosure had good industrial application prospect in lysine production.

### Example 4: Modification of gluconate repressor GntR1 to improve lysine yield

In order to further improve the lysine yield, the present disclosure further introduced an R102A mutation (the amino acid sequence after mutation was as set forth in SEQ ID NO: 13) of the gluconate repressor GntR1 (NCBI-Protein ID: BAB99920, Gene No. Cgl2527 in *Corynebacterium glutamicum* ATCC 13032 genome GenBank: BA000036.3 in NCBI) into the SCgL42 (pEC*-MsLysO*) strain, and evaluated the effect of this mutation on lysine production. A CRISPR/Cas9 single-plasmid genome editing system was used to construct a mutant strain in which an R102A mutation (the codon was mutated from CGT to GCC) was introduced in the *gntR1* gene on the genome of the SCgL42 strain.

The mutant strain was specifically constructed as follows:
1) Construction of editing plasmids. The upstream and downstream homologous arms were respectively amplified using *gntR1*-UF and *gntR1*-UR as well as *gntR1*-DF and *gntR1*-DR as primers respectively and using the *Corynebacterium glutamicum* ATCC 13032 genome as a template. Two plasmid skeleton fragments and one gRNA fragment were respectively amplified using Cas-1 and Cas-2, Cas-3 and CasgRNA-*gntR1* as well as gRNA-*gntR1* and gRNA-2 as primers respectively and using pCas9gRNA-*ccdB* (CN112111469A, which was incorporated herein by reference) as a template. The above upstream and downstream homologous arm fragments and plasmid skeleton fragments were ligated by cloning via Vazyme One-Step Cloning Kit to obtain the recombinant plasmid pCas9gRNA-*gntR1*^{R102A}.
2) Competent cells were prepared from the SCgL42 strain, in which 1 to 2 µg of pCas9gRNA-*gntR1*^{R1021} plasmid was electro-transformed under the electric shock conditions of 2500 V and 5 ms. The cells were immediately added to 1 mL of TSB medium preheated at 46°C, subjected to thermal shock at 46°C for 6 min, incubated at 30°C for 3 h, coated on a TSB plate in which 5 µg/mL of chloramphenicol and 0.05 mM IPTG were added, and cultured at 30°C until monoclones were obtained. The *gntR1*-C1/C2 was used as a primer for amplification to verify the mutants and obtain the correct mutant according to the sequencing results. For the correct mutant clones, the plasmid loss was carried out as follows: the clones were cultured overnight at 30°C in a TSB liquid medium without the resistance; then monoclones were streaked on a TSB solid medium plate without the resistance; subsequently, the grown monoclonal bacteria were streaked on two solid plates (TSB+5 µg/mL of chloramphenicol and TSB) respectively, and cultured at 30°C for 24 h. The resulting strain that did not grow on the chloramphenicol-resistant plate but could grow on the TSB plate was the mutant from which the plasmid was lost, *i.e.* the strain SCgL42*gntR1*^{R102A}. SCgL42*gntR1*^{R102A} was then used to prepare competent cells and the pEC-*MsLysO* plasmid was introduced to obtain the SCgL42*gntR1*^{R102A} (pEC-*MsLysO*) strain. The sequences of the primers as used above were as shown in Table 2.

**Table 2**

| Primers | Nucleotide Sequences | SEQ ID NO. |
|---|---|---|
| *gntRl-DF* | AATCTCGTGATGGCAGGTTGACGCTGAGAAGGACT ACGATTCC | SEQ ID NO: 14 |
| *gntRl-DR* | CCAGCGAATGATGGACTTATC | SEQ ID NO: 15 |
| *gntRl-DF* | ATAAGTCCATCATTCGCTGGGCCCTCAATGACGAA GGTCAGCGTG | SEQ ID NO: 16 |
| *gntRl-DR* | CAGTTATTGGTGCCCTTCGACGTTGAAGCTCTTAG CGATATTGC | SEQ ID NO: 17 |
| Cas-1 | TCGAAGGGCACCAATAACTGC | SEQ ID NO: 18 |
| Cas-2 | CTTTTACTTTCACCAGCGTTTCTG | SEQ ID NO: 19 |
| Cas-3 | AACGCTGGTGAAAGTAAAAGATGC | SEQ ID NO: 20 |
| CasgRNA-*gntR 1* | AGCTGTTTCAAAGCGCGCATTGAATTACACTGTAC CTGTTGCGTC | SEQ ID NO: 21 |
| gRNA-*gntR1* | ATGCGCGCTTTGAAACAGCTGTTTTAGAGCTAGAA ATAGCAAG | SEQ ID NO: 22 |
| gRNA-2 | CAACCTGCCATCACGAGATTTTC | SEQ ID NO: 23 |
| *gntR1*-C1 | TTGACCGTGACTACACCCCAC | SEQ ID NO: 24 |
| *gntR1-C2* | ATCCCATCCCCACCGAATCTG | SEQ ID NO: 25 |

The SCgL42 *(pEC-MsLysO)* and SCgL42*gntR1*^{R102A} *(pEC-MsLysO)* strains were subjected to the lysine fermentation test by the same method described in Example 3, respectively. The results were shown in Table 3. Introduction of an R102A mutation to the *gntR1* gene of the SCgL42 (pEC-*MsLysO*) strain could further improve the L-lysine yield.

**Table 3 Effect of GntR1 Mutation on Lysine Production**

| Strains | Lysine Yield (g/L) |
|---|---|
| SCgL42 (pEC-*MsLysO*) | 7.7±0.2 |
| SCgL42*gntR1*^{R102A} (pEC-*MsLysO*) | 8.4±0.1 |

All technical features disclosed in the present specification may be combined in any combination manner. Each of the technical features disclosed in the present specification may be substituted by other features having the same, equivalent or similar effects. Thus, unless specifically stated, each of the features disclosed herein is only an example of a series of equivalent or similar features.

In addition, with the foregoing descriptions of the present disclosure, the key features of the present disclosure become apparent to a person skilled in the art. Many modifications may be made to the invention without departing from the spirits and scope of the present disclosure, so as to adapt the invention to various application purposes and conditions. Therefore, such modifications are also intended to fall within the scope of the appended claims.
SEQ ID NO: 1 
MYSGLLIILL PLLFGYLLPL RAKKVLQTIN QLLSWMVYVI LFLMGISLAF LDNLGSNLLL 60
IFRYAAVFFV CIIAANALSL WLWGKRDVWP GKFPQEAPPS RLYMILDSLK LCLVVFGGFL 120
LGLTQWPGLI HASEGSEYAL ILLLFFVGIQ LRNSGMTLRQ IVLNRRGMSI ALVVGVSALA 180
GGLLAARVLS LPLKTGLAIA SGYGWYSLSG ILLTDALGPV IGSAAFFNDL ARELLAIMLI 240
PALTRRNRAC ALGLCGATSM DFTLLVLQRS AGVDMVPAAI VHGFLLSLVA PVFMALFSS 299
SEQ ID NO: 2 
MLSGLLLVLL PLLLGYLVPV HHTGAMDVIN RSVNAAIYVI LLLMGVGLAG LDDLASQLSR 60
MGSQALLLFL LLSACNLAAL AWLARRLRMR IEPGRSVADA PTSKLAALAG SLALVGVVIG 120
GVLTGLVASG LSAGEGFFSG AETLAEGVLY LLLALIGCQL RNSGMPLRQI LLNRRGLAIA 180
ATLAASSLVG GLVAAPLLSL AWNEGLALAA GFGWYSLSGI LIGDGLGAQM GGVAFFNDLL 240
RELAAFVLIP LTIRRHAPLA IGYGGATAMD FTLPVIQQHG GVACVPVAVV SGFLLSLLSP 300
PLILFFLSL 309
SEQ ID NO: 3 
MSLESLVILL PLFAGYLIPV TRPALLTGID KLLSAIVYLI LALMGLSLAQ VDNLAGELSS 60
ILQLVALLIG LSLVLNLALL PLVDRRWPMA LTLNNGHQPL GKMMLESLKL AAVVVGGVAL 120
GLVLPVGHQA IESGANAALM LLLGLIGIQL RASNLGLRAI LLNPRGLAIA AIVAVTSLLA 180
GALTALIAGL PVIHGITLAA GFGWYSLSGI LITDGLGPVM GSVAFLFDLS RELIAIVIIP 240
MLMRRSPASA IGYGGATAMD FTLPVLQQTG GAPVVPVAIV SGFLLSLASP VLIPFLLSLA 300
SEQ ID NO: 4 
MLASALLILV PLFVGFAFHV ENRRVLSTIN HSLEALVYLI LALLGISLGQ LEGLAEQLNR 60
MTGQVLVLVF GLLIANVAGL WLWHRWQPMT LAAAEDSQGN VGYRRLFLGA AKPLLAVIAG 120
MALGHWLLPA VSWIEDVASG ALMLLLLLIG LQLRNAGLSL RRLLMNRQGL GIAVTLALTS 180
LLAGLAIAPF YDLPLMHVLA LTSGFGWYSL SGIVVGDALG PAWGGVAFLN DVLREIIALV 240
LIPLIIARRP AMAIGYGGAT AMDFTLPIIR RSGGLETVPV AIASGFILSF LSPVLMGLFL 300
AG 302
SEQ ID NO: 5 
MTALSSLMPI VGALAGGWAA SHVLPRTLRH WLIRLIGPLV WLLLFLIGME FGAVLSSAEA 60
LGSALYTAGV LALFTTLVPC LLIWLLIRRT RGSATAPRRE ANTATFWQPV KECGIALSMV 120
VLGGLAFTWM PASIAHAHPL PSSWTLLLVL IVLVGIDLGQ VDLKGRLWSF KAFAIPLLVV 180
LGSLLGACAA AWATGENLQV ALALASGFGW FSLSSVMIGE ALGQNYGTIA LMTDLLRELL 240
AIVILYMAGM HYPLPAVGSA GATALDTTLP IVKQTCAPDI VPLALVSGFI LTVLAPFMIM 300
VFLAH 305
SEQ ID NO: 6 
MNNALWATVP ILLALVLGFG LGQYVNDQYR NYVSRCVGIL VWVLLFSLGA EFGSVLGNPQ 60
LAARSLFVGI TFAMTCTLAT WLLIHLLTAT SDRKAAFRLT ELLSSLVEGL KSCTVTLSIV 120
TLGAIYAHFP IDWITSIWKP TLGDMICVLV FLVGIELAKL NLGRDLFTPR SITVPLITVV 180
GTLSGGLLAA WIVDEPLKIG LVLASGFGWM SLSSAMVSET LGKSYGSMVL IADLLRELLA 240
ITLLYIFGMK APLACIGATG VTSLDSTLPI VRVTCPEEVI PYAVVSGLIL TVVSPLFIAF 300
FLTL 304
SEQ ID NO: 7 
atgtattcgg gattattaat tattttattg ccattactgt ttggctatct gttgcctttg 60
cgcgcgaaaa aagtcctaca gacaattaat cagctattga gttggatggt gtacgtcatt 120
ctatttttaa tgggaattag cctggctttt ctggataacc tcggcagcaa tctgttactg 180
atttttcgtt acgccgccgt tttctttgtg tgcatcattg ccgccaatgc cttgtcgcta 240
tggctgtggg gaaaacgtga tgtctggccg ggaaaatttc cccaggaagc gccgccttcc 300
cgcctgtata tgattctgga ttcgttaaag ctctgtctgg tggtatttgg cggttttctg 360
ctggggttga cgcaatggcc tggtttgatc catgccagtg aaggcagcga atacgcgctg 420
attctgttgt tgtttttcgt cggaatacag ttacgcaaca gcggaatgac gctacgtcaa 480
atcgtgttga atcgacgggg aatgtcgatt gcgttggttg tcggcgtcag tgcgctggca 540
ggcggtttac tcgccgccag ggtgctcagc ctgccgctca aaaccgggct ggcgatagca 600
tccggctatg gctggtattc gttatccggc attctgctga ccgacgcctt aggcccggtg 660
atcggcagcg cggccttttt caacgatctg gcgcgagaac tgttagccat catgttgatt 720
cccgcgctga cacgacgcaa ccgcgcctgt gcgctcggac tgtgcggcgc gacgtcgatg 780
gatttcacgt tgctggtgtt gcaacgcagc gccggggtcg atatggtgcc ggcggccatt 840
gtgcacggtt ttctattgag tcttgtcgcg ccggtattca tggcgctatt ttcctcctga 900
SEQ ID NO: 8 
atgctgagcg gtctgctgct cgtcctgctg ccgttgctgc tgggctatct ggtgccggtg 60
catcacaccg gcgcaatgga tgtcatcaac cgcagcgtca acgccgccat ctacgtgatc 120
ctgctgttga tgggtgtggg cctggccggc ctcgacgacc tggccagcca gctctcgcgc 180
atgggcagcc aggcactgct gctgttcttg ctgctctccg cctgcaacct ggcggcattg 240
gcctggctgg cccgacgcct gcggatgcgc atcgaacccg gccgctcggt ggccgacgcg 300
ccgaccagca agctcgcggc cctggccggc tcgctggcac tggtgggggt ggtgatcggc 360
ggcgtgctga cggggctggt cgccagcggc ctgagtgcgg gtgaagggtt tttcagcggt 420
gccgaaacgc tggccgaagg tgtgctctac ctgctgttgg cgctgatcgg ctgccagctg 480
cgcaactccg gcatgccact gcgccagatt ctgctcaacc gccgcggcct ggccattgcc 540
gccaccctgg cggccagttc gctggtgggt gggctggttg ccgcgccact gctctccctc 600
gcctggaacg aagggctggc gctggctgcc ggcttcggct ggtactcgct ttccggcatc 660
ctgatcggcg acgggctggg cgcccagatg ggcggcgtgg ccttcttcaa cgacctgctg 720
cgcgaactgg cagccttcgt gctgatcccg ctgaccatcc gccgccacgc cccgctggcc 780
atcggctacg gcggcgccac ggccatggat ttcaccctgc cagtgattca gcagcacggt 840
ggcgtggctt gcgtacccgt cgccgtggtc tcgggctttc tgctctcgct gctatcgccg 900
ccgttgatcc tgtttttcct gtcattgtga 930
SEQ ID NO: 9 
atgtccctag aatccctggt tattcttctc cccctgtttg ccggttacct gattccggta 60
acccgcccgg ccctgctgac cggcatcgac aagctgctga gcgccatcgt ctatctgatt 120
ctggcgttga tggggctgtc actggcccag gtggacaacc tggccggcga gctgagcagc 180
atcctgcagt tagtggcgct gctgatcggt ttgagcctgg tactgaacct ggcgctgttg 240
ccgctggtgg atcgacgctg gcccatggct ctgaccctga acaacggcca tcagccactg 300
ggcaagatga tgttggaatc gttgaaactg gcggccgtgg tggttggcgg ggtggcgttg 360
gggctggtgc tacccgtcgg ccatcaggcc attgagtctg gcgccaacgc cgcgctgatg 420
ctgctgttgg ggctgatcgg gattcagctg cgggcgtcca acctggggct acgggcgatt 480
ctgctcaacc ccaggggact ggccatcgcc gccattgtcg ccgtcacctc actgctggcc 540
ggcgccctga ccgccctgat cgccgggctg ccggtgattc acggcatcac cctggcggcg 600
ggctttggct ggtactccct cagcggcatc ctgatcaccg acggcctggg gccggtaatg 660
ggctcggtgg ccttcctgtt cgacctcagc cgcgagctga tcgccattgt gattattccg 720
atgctgatgc gccgctcgcc ggcgtccgcc atcggctacg gcggcgccac ggccatggac 780
ttcaccctgc cggtactgca gcagaccggc ggtgccccgg tggtgccagt ggccattgtg 840
tccggcttcc tgctgagcct ggccagcccg gtgctgatcc ccttcctgct gtcgctggcg 900
taa 903
SEQ ID NO: 10 
atgcttgcca gcgcgctgct gatcctggtg cctctctttg tcggttttgc cttccacgtc 60
gagaatcgcc gtgtactgtc aacgatcaac cattcgctgg aagcgttggt gtatctcatt 120
ctggctctgc tcggcatcag cctgggccag cttgagggat tggcggagca attgaaccgg 180
atgaccggtc aggtgctggt cctggttttc ggattgctga tcgccaacgt tgccggcctc 240
tggttgtggc atcgctggca gccaatgacg ctggcagcgg ccgaggacag tcagggcaat 300
gtgggttacc ggcgtttgtt cctcggtgcg gcaaaaccac tgctggcggt gattgccggc 360
atggcgctgg gccactggct gttgcccgca gtgagctgga tcgaggacgt ggccagcggg 420
gcgctgatgc tcctgctgct gctgatcggc ctgcagttgc gtaatgccgg tctgtccctg 480
cggcgcctgc tgatgaaccg gcagggtctg gggattgctg tcaccctggc gctgacctcg 540
ctgctggcgg gcctggcgat cgcgccgttt tatgatctgc ccctgatgca tgtgttggcg 600
ctgacgtcgg gctttggctg gtattccctg tcgggcatcg tggtggggga tgcgttgggg 660
ccggcctggg gcggcgtggc gtttctcaac gatgtcctgc gggaaatcat cgccctggtg 720
ctgattccat tgatcatcgc ccggcggcca gccatggcca tcggctacgg cggcgccacc 780
gccatggact tcaccctgcc gatcatccgc cgcagcggcg gcctggaaac cgtgcccgtc 840
gcgatcgcct cgggtttcat cctgtccttc ctgtcgcccg ttctgatggg cctcttcctc 900
gccggctga 909
SEQ ID NO: 11 
atgaccgccc tctcctctct gatgccgatt gtcggcgcgc tcgccggcgg ctgggccgcc 60
agccatgtct tgccgcgcac gctgcggcac tggctgatac ggttgatcgg cccgctggtc 120
tggctgctgc tgtttctgat cggcatggaa ttcggcgccg tgctctcgtc ggccgaagcg 180
ctgggcagcg cgctctacac agccggcgta ctggccttgt tcaccaccct ggtgccatgc 240
ctgctgatct ggctgctgat acggcgcacc cgaggcagcg caacggcgcc gcgccgcgaa 300
gccaataccg ccacgttctg gcaaccagtg aaagaatgcg gcattgcctt gtccatggtg 360
gtgctcgggg ggctggcctt tacctggatg cccgccagca ttgcgcacgc ccatccgttg 420
ccatcgagct ggacgctgct gttggttctg atcgtactgg tcggcatcga cctgggccag 480
gtcgacctga aaggccggct ttggtcattc aaggccttcg ccatcccgct gctggtcgtg 540
ctcggctcgt tgctgggcgc ctgcgctgcc gcctgggcga ccggtgaaaa tcttcaggtc 600
gcgcttgcgc tggcgtcggg cttcggctgg ttttcgctgt ccagcgtcat gattggcgag 660
gccctgggcc agaactacgg caccatcgcc ctgatgaccg acttgctgcg tgagctgctg 720
gccattgtca tcctctatat ggccggcatg cattatccgc tgcccgccgt cggcagcgcg 780
ggcgctaccg ccctggacac caccttgccc atcgtcaagc agacctgcgc gccggatatt 840
gttcccctgg cactggtgag cggtttcatc ctgaccgtgc ttgcgccctt catgatcatg 900
gtgttcctgg cgcactga 918
SEQ ID NO: 12 
atgaacaacg cactatgggc gactgtgcct attcttttgg cacttgtcct tgggtttggg 60
ctcggccaat acgtaaacga tcagtatcga aactacgtct cacgatgcgt cgggatactg 120
gtctgggtgc tacttttttc gctgggtgca gagtttggta gcgtgctcgg caatccgcag 180
ttggcagcaa gatctctgtt tgtcggcatc acctttgcca tgacctgtac ccttgccact 240
tggctgttga ttcatttgtt gacagctacg agtgatcgca aagctgcctt caggctgacg 300
gaactgctca gttctcttgt cgaaggtttg aagagttgca ccgtcacttt gtccatcgtc 360
acgttgggag caatctatgc tcatttccca attgactgga tcacctcaat ctggaagccc 420
acgctgggtg acatgatttg cgtgctggtt tttctagtgg gtattgagct ggctaagttg 480
aatttgggcc gcgatctctt cactcccaga tccatcaccg ttcccctcat cacggtagtg 540
ggtacattat ccggcggact tctggctgcc tggatagtcg atgaaccact caagatcgga 600
ctggtgcttg cttcggggtt tggctggatg agcttgtcca gtgcaatggt cagcgaaaca 660
ctgggcaaat cctacggctc gatggttttg attgcggacc tcctgcgaga actcctggct 720
atcaccctgc tttacatttt cggcatgaaa gcgcctctgg cctgcattgg cgccactggc 780
gtcacttcgc tggactcaac cttgccgatt gttcgcgtga catgccccga agaagtcatt 840
ccctacgctg ttgtcagcgg cctgattctg acagtggttt cccctctgtt tatcgctttt 900
ttcctgacgc tatga 915
SEQ ID NO: 13

MTPANESPMT NPLGSAPTPA KPLLDSVLDE LGQDIISGKV AVGDTFKLMD IGERFGISRT 60
VAREAMRALE QLGLVASSRR IGITVLPQEE WAVFDKSIIR WALNDEGQRE GQLQSLTELR 120
IAIEPIAARS VALHASTAEL EKIRALATEM RQLGESGQGA SQRFLEADVT FHELILRYCH 180
NEMFAALIPS ISAVLVGRTE LGLQPDLPAH EALDNHDKLA DALLNRDADA AETASRNILN 240
EVRSALGTLN 250

## Claims

1. A recombinant microorganism, wherein the recombinant microorganism has an enhanced activity and/or expression level of a lysine efflux protein as compared to a wild-type microorganism; and the lysine efflux protein is at least one selected from the group consisting of (i) to (iv):
(i) a polypeptide comprising an amino acid sequence as set forth in SEQ ID NO: 4;
(ii) a polypeptide mutant comprising an amino acid sequence as set forth in SEQ ID NO: 4, the polypeptide mutant having one or more amino acids substituted, repeated, deleted or added at one or more positions of the sequence as set forth in SEQ ID NO: 4, and having an activity of the lysine efflux protein;
(iii) a polypeptide comprising an amino acid sequence as set forth in SEQ ID NO: 6; and
(iv) a polypeptide mutant comprising an amino acid sequence as set forth in SEQ ID NO: 6, the polypeptide mutant having one or more amino acids substituted, repeated, deleted or added at one or more positions of the sequence as set forth in SEQ ID NO: 6, and having an activity of the lysine efflux protein.

2. The recombinant microorganism according to claim 1, wherein the lysine efflux protein is encoded by a polynucleotide shown in any one of (v) to (vii):
(v) a polynucleotide encoding a polypeptide of the amino acid sequence as set forth in SEQ ID NO: 4 or SEQ ID NO: 6;
(vi) a polynucleotide comprising a nucleotide sequence as set forth in SEQ ID NO: 10 or SEQ ID NO: 12; and
(vii) a polynucleotide having at least 90%, optionally at least 95%, preferably at least 97%, more preferably at least 98%, most preferably at least 99% to the sequence as set forth in SEQ ID NO: 10 or SEQ ID NO: 12 and encoding a polypeptide having an activity of the lysine efflux protein.

3. The recombinant microorganism according to claim 1 or 2, wherein the recombinant microorganism is derived from a microorganism of *Escherichia, Erwinia, Serratia, Providencia, Enterobacteria, Salmonella, Streptomyces, Pseudomonas, Brevibacterium* or *Corynebacterium;*
optionally, the recombinant microorganism is derived from *Corynebacterium glutamicum* or *Escherichia coli;*
preferably, the recombinant microorganism is derived from *Corynebacterium glutamicum* ATCC 13032, *Corynebacterium glutamicum* ATCC 13869, *Corynebacterium glutamicum* ATCC 14067 or a derivative strain of any one thereof.

4. The recombinant microorganism according to any one of claims 1 to 3, wherein the recombinant microorganism contains a mutated gluconate repressor GntR1; preferably, the mutation is a mutation of arginine at position 102 to alanine.

5. A method for constructing the recombinant microorganism according to any one of claims 1 to 4, comprising a step of enhancing an activity of a lysine efflux protein in a microorganism and/or a step of enhancing an expression level of a lysine efflux protein in a microorganism; wherein the lysine efflux protein is at least one selected from the group consisting of (i) to (iv):
(i) a polypeptide comprising an amino acid sequence as set forth in SEQ ID NO: 4;
(ii) a polypeptide mutant comprising an amino acid sequence as set forth in SEQ ID NO: 4, the polypeptide mutant having one or more amino acids substituted, repeated, deleted or added at one or more positions of the sequence as set forth in SEQ ID NO: 4, and having an activity of the lysine efflux protein;
(iii) a polypeptide comprising an amino acid sequence as set forth in SEQ ID NO: 6;
(iv) a polypeptide mutant comprising an amino acid sequence as set forth in SEQ ID NO: 6, the polypeptide mutant having one or more amino acids substituted, repeated, deleted or added at one or more positions of the sequence as set forth in SEQ ID NO: 6, and having an activity of the lysine efflux protein.

6. The method according to claim 5, wherein the method further comprises a step of mutating a gluconate repressor GntR1 of the recombinant microorganism; preferably, the mutation is a mutation of arginine at position 102 to alanine.

7. Use of the recombinant microorganism according to any one of claims 1 to 4, or a recombinant microorganism constructed by the method according to claim 5 or 6 in the production of lysine.

8. A lysine producing strain, wherein the lysine producing strain is the recombinant microorganism according to any one of claims 1 to 4, or a recombinant microorganism constructed by the method according to claim 5 or 6.

9. The lysine producing strain according to claim 8, wherein the lysine producing strain is derived from a microorganism of *Escherichia, Erwinia, Serratia, Providencia, Enterobacteria, Salmonella, Streptomyces, Pseudomonas, Brevibacterium* or *Corynebacterium;*
optionally, the lysine producing strain is derived from *Corynebacterium glutamicum* or *Escherichia coli;*
preferably, the lysine producing strain is derived from *Corynebacterium glutamicum* ATCC 13032, *Corynebacterium glutamicum* ATCC 13869, *Corynebacterium glutamicum* ATCC 14067 or a derivative strain of any one thereof.

10. A method for producing lysine, comprising a step of producing lysine by fermenting the recombinant microorganism according to any one of claims 1 to 4, the lysine producing strain according to claim 8 or 9, or a recombinant microorganism constructed by the method according to claim 5 or 6;
optionally, the method further comprising a step of isolating the lysine from a reaction solution for producing the lysine by fermentation.
